# EUROPEAN PATENT SPECIFICATION

(11) **EP 1 689 452 B1**
(45) Date of publication and mention of the grant of the patent: **25.12.2013**
(21) Application number: 04800374.3
(22) Date of filing: 24.11.2004
(51) Int. Cl.: A61L 15/28, A61L 15/44

(54) **ABSORBENT ARTICLE CONTAINING A SKIN-CARE AGENT**
SAUGFÄHIGER ARTIKEL MIT EINEM HAUTPFLEGEMITTEL
ARTICLE ABSORBANT CONTENANT UN AGENT DE SOINS DE LA PEAU

(30) Priority: 24.11.2003 SE 0303123
(43) Date of publication of application: 16.08.2006
(73) Proprietor: SCA Hygiene Products AB, 405 03 Göteborg (SE)
(72) Inventor: RUNEMAN, Bo, S-41708 Göteborg (SE); FORSGREN-BRUSK, Ulla, S-435 43 Pixbo (SE)
(74) Representative: Romare, Laila Anette
(86) International application number: PCT/SE2004/001713
(87) International publication number: WO 2005/049100

(56) References cited:
- WO-A2-99/56681
- US-A- 5 643 582
- US-B1- 6 207 194
- US-B1- 6 416 779
- US-B1- 6 475 197
- US-B1- 6 649 806
- CASWELL M. ET AL.: 'Comparison of the moisturization efficacy of two vaginal moisturizers: pectin versus polycarbophil technologies' JOURNAL OF COSMETIC SCIENCE vol. 53, 2002, pages 81 - 87, XP008015097

## Description

### TECHNICAL FIELD

The invention relates to an absorbent article, such as a sanitary towel, panty liner, tampon, diaper, interlabial product or incontinence protection, which is provided with a skin-care agent which is intended to be transferred from the absorbent product to the user. In particular, the invention relates to an absorbent article which is provided with a skin-care agent which is intended to be transferred to mucous membranes and skin tissues in the lower abdomen of a woman. The skin-care agent to which the invention relates contains pectin.

### PRIOR ART

An absorbent article contributes to increasing the dryness in the areas where its absorbent part is in contact with the user since it is intended to take up and absorb liquid. Dehydration of skin and mucous membranes can lead to smarting and discomfort and to impaired protection against infection. This problem is especially common in older, postmenopausal women, whose mucous membranes frequently become atrophied, fragile and dry as a consequence of diminished oestrogen production. Their vaginal microflora and environment change drastically from having been dominated by a protective flora of lactobacilli and an acid pH of approximately 3.5-4.5 to a pronounced presence of potentially pathogenic bacteria, first and foremost E. coli, from the intestine and the area of the anus, and a pH of approximately 5-6. Urinary tract infections, which are frequently caused by E. coli bacteria, occur more frequently in older women. Incontinence also contributes to an increase in the risk of urinary tract infections. The article "A controlled trial of intravaginal estriol in postmenopausal women with recurrent urinary tract infections" (Raul Raz and Michael E. Stamm, Vol. 329, No. 11, pages 753-756 The New England Journal of Medicine) describes these problems in more detail.

The problem of dehydrated and atrophied vaginal mucous membranes is consequently known and a number of medical preparations are available for treating it. For example, US 6,346,267 describes a moisture-preserving composition which contains a mixture of phytooestrogen and herbs. US 5,474,768 also describes a moisture-preserving composition which is water-based and contains a bioadhesive substance and a consistency-imparter. The described substance can expediently be applied to the mucous membranes by spraying or rubbing or as suppositories.

A number of patent specifications have also disclosed how absorbent articles have been provided with oils and creams for the purpose of counteracting dehydration of the skin and mucous membranes. US 6,416,779 describes, for example, how a tampon can be used for transferring a therapeutic substance to the vagina, and US 6,475,197 describes how a skin-care agent is transferred from a diaper, sanitary towel or the like to the user.

The use of pectin in skin-proximal products is described in the patent literature. GB 696,608 reports that pectin is used as a carrier for bacterial inhibitors, and US 4,813,942 uses pectin for its pH-reducing properties. EP 343,807 uses pectin in a composition for its adhesive properties when it is moist. These 3 documents specify wound-care products as the area of application and nor do any of them indicate that the pectin should be transferred to the skin. DE 38 34 797 uses pectin in an incontinence protection. The pectin is present in the form of an absorbent in the core of the product for the purpose of absorbing liquid and functioning as an odour inhibitor. There is nothing in DE 38 34 797 which indicates that pectin is to be used as a skin-care agent and, even less, that the intention is for the pectin to be transferred to the skin.

The article "Comparison of the moisturization efficacy of two vaginal moisturizers: pectin versus polycarbophil technologies" (Michael Caswell and Michael Kane; J. Cosmet, Sci., 53, 81-87 (March/April 2002)) states that a pectin-based substance exhibits just as good an effect in regard to moisturizing vaginal mucous membranes as does a preparation which contains a polycarbophil. These preparations are reported to have just as good an effect on the mucous membrane as does oestrogen. Scientific evidence consequently exists for pectin having an effect as a skin-care agent on vaginal mucous membranes.

### DISCLOSURE OF THE INVENTION

The invention is intended to solve the problem of dehydrated and irritated tissues, first and foremost vulva skin and mucous membranes, in the lower abdomen of women, which problem can occur on the body parts which are in contact with an absorbent article.

An object of the invention is to produce an absorbent article, such as a sanitary towel, panty liner, tampon, diaper, interlabial product or incontinence protection, which possesses improved skin-care properties.

Another object of the invention is to produce an absorbent article which contributes to a healthier environment, involving good protection against infection, in the lower abdomen of women.

Yet another object of the invention is to produce an absorbent article which contributes to remedying problems of atrophied, fragile and dry mucous membranes when using sanitary towels, tampons, incontinence protection and similar products.

It is furthermore an object of the invention to produce an absorbent article which contributes to retaining a low pH in the lower abdomen of women.

Another object of the invention is to produce an absorbent article which is provided with a skin-care agent for counteracting and alleviating skin irritations, first and foremost in infants and older persons, when using diapers or incontinence protection.

This is achieved by means of an absorbent article, such as a sanitary towel, panty liner, tampon, diaper, interlabial product or incontinence protection, which is provided with a skin-care agent which contains pectin. The skin-care agent is intended to be transferred from the absorbent product to the user, that is to say that the skin-care agent containing pectin is at least partially emitted by the product, and transferred to the user, during use. As a result of its moisture-preserving property, the pectin will counteract the negative effects which arise in connection with the dehydration of skin tissues and mucous membranes. The invention is regarded as being particularly well-suited for tampons, interlabial products, sanitary towels/incontinence protection provided with humps or similar products which are at least partially intravaginal and directly abut the woman's mucous membranes and thereby contribute in having a dehydrating effect on said mucous membranes.

In the application, a skin-care agent is understood as meaning a composition which contains a substance which affects the skin and can be used for preventing, counteracting or alleviating different types of irritation or damage which can occur on skin tissues or mucous membranes. These substances can, for example, be used for counteracting dehydration or regulating the pH and can have antibacterial or antiinflammatory properties.

In addition, the invention is intended to encompass the type of absorbent articles which are intended to be regularly used for absorbing menstruation liquids, urine and faeces.

A further advantage of pectin is the possibility it provides of influencing the pH in the lower abdomen of the user. A problem which is particularly frequent in postmenopausal women is that the pH in the vagina increases as a result of a decrease in oestrogen production. By means of choosing a pectin which has a desired pH value, pectin can thus be employed to function as an acidifying agent in addition to counteracting dehydration. A lower pH in the vagina and on vulva skin provides the opportunity to establish a more favourable bacterial flora. The antibacterial properties of the pectin also enable it to be used in incontinence protections and diapers in order, for example, to avoid or impede infections connected with pressure sores (bed sores) or diaper-induced skin irritation (contact dermatitis).

Pectin is a polysaccharide having a mean molecular weight of 20 000-400 000 u. The polysaccharide is in the main composed of galacturonic acid units, which can be esterified to varying degrees. Pectin is naturally present in the cell walls of all plants and functions as a binding agent. A common source for extracting pectin is citrus peel. Naturally occurring pectin is an integral part of the complex structure which imparts stability to a plant. In this case, the pectin consists of several different types of neutral sugar molecules, which are present in a complex, defined pattern which contains sequences of homogalacturonic acid which are sandwiched with sequences of neutral sugar molecules such as rhamnose, galactose, arabinose and other sugar types which describe a well-branched structure. When pectin is extracted, a large part of the branched structure usually disappears and a relatively straight chain, the majority of which consists of galacturonic acid molecules, remains. Pectin is usually defined as a polymer which contains more than 65% galacturonic acid. The acid groups can either be free or esterified. When pectin is extracted, more than 50% of the galacturonic acid is usually esterified, and the pectin is then classified as being high-methyl ester pectin, and when the pectin has a degree of esterification of less than 50%, it is consequently classified as being low-methyl ester pectin.

While pectin which is intended to be used in the invention does not have any critical limit as regards its molecular weight, it preferably has a molecular weight of between 50 000 and 150 000 u. However, pectins having a molecular weight outside this range also exhibit similar properties and can be used within the context of the invention. The degree of esterification is regarded as being of secondary importance for its function in this connection and it is possible to use pectins having widely different degrees of esterification. On the other hand, the degree of amidation is of importance in the present invention. The degree of amidation affects the pH of the pectin, and no amidation at all, is desirable for producing a low pH.

An experiment in which pH measurements have been carried out on skin after the skin has been covered with pectin films is described below. Pectin, of the brand GENU® pectin (citrus) type USH-H (manufacturer: CP Kelco, Denmark), was dissolved in 80°C tap water while stirring vigorously (1 000 rpm) to give a 2% solution. The solution was dried overnight in flat plastic dishes, resulting in a thin film forming on the bottom. Samples of 1 x 4 cm in size and weighing approx. 15 mg were cut out. These sample pieces were placed for one hour on the forearms of two experimental subjects. The sample pieces, as well as corresponding skin surface without sample, were covered with PE film (not vapour-permeable). The pH was measured before placing the samples and after removing them, after having slightly moistened the skin with a few drops of deionized water. A Courage+Khazaka, type PH 900 pH meter was used in the experiment. The results of the experiment are recorded in Table 1.

**Table 1. Results of measuring the pH of the skin**

| **Experimental subject** | **Pectin film** | **Prior skin pH** | **Skin pH after 1h** |
|---|---|---|---|
| **1** | - | 4.7 | 4.9 |
| **1** | yes | 4.7 | 4.4 |
| **2** | - | 5.6 | 5.7 |
| **2** | yes | 5.6 | 5.0 |

Table 2 lists the pectin which was used together with a number of other pectins which can also be expediently used. However, the invention is not to be regarded as being restricted to the pectins which are mentioned here.

**Table 2. Different pectin grades, GENU® pectin (citrus) from CP Kelco, Copenhagen, Denmark.**

| **Type** | **pH of 1 % aqueous solution** | **Degree of esterification** | **Amidated** |
|---|---|---|---|
| **(reference) X-916-02** | 4.0-5.0 | low | yes |
| **(reference) X-917-02** | 4.0-5.0 | low | yes |
| **X-920-02** | 2.5-3.1 | low | - |
| **USP-100** | 3.6-4.1 | high (60%) | - |
| **USP-H** | 3.6-4.4 | high (72%) | - |

The pectin can be present in the product, on its own or together with other substances such as consistency imparters, odour inhibitors and other active substances. The skin-care agent containing pectin can be present in the form of a gel, film, fibre, liquid, cream, powder, emulsion, paste, etc. The essential point is that the pectin is an integral part of a carrier system which efficiently effects a transfer of the pectin to the user while at the same time interfering as little as possible with the absorptive ability of the product. The system which is used depends, inter alia, on the type of absorbent product which is intended, the quantities which are to be released, how rapidly the transfer is to take place or whether the intention is for the pectin to act together with other substances. It is even conceivable to use the pectin on its own as a type of carrier of active substances, which the pectin can release when it has adhered to the skin or the mucous membranes.

The location of the skin-care agent on the article is important for ensuring that the agent can be transferred. The skin-care agent must be located in the product such that it is transferred to the user when the product is being employed. While the skin-care agent containing pectin can advantageously be applied to different sites in the product, it has to be located such that it comes into contact with the skin tissue or mucous membrane tissue of the user in order to be able to be transferred to the latter. When it is applied to an absorbent article which has a liquid-permeable surface layer, a liquid-impermeable rear-side layer and an absorbent body in between, the skin-care agent is advantageously applied to the surface layer, which faces the user. It is also conceivable to apply the skin-care agent in the surface layer, for example by means of allowing it to be a part of the surface material or by locating it between some layers if the surface layer is a laminate. The skin-care agent can also be located directly below the surface layer. However, the further the agent is located from the surface layer and the skin of the user, the more difficult it then is to provide for satisfactory transfer of the skin-care agent to the user. When the absorbent article is in the form of a diaper or an incontinence protection, having a belt-like fastening around the waist, or of a panty-like model, the skin-care agent can, for example, be applied to the belt, or to the side panels belonging to the panty-like models, in order to counteract skin irritations in the region of the waist.

While, in the case of tampons and similar absorbent articles, the skin-care agent is advantageously applied to the outside surface, which faces the user, it can also be located in, or directly below, the outside surface. These examples only describe some of the sites to which the skin-care agent can be applied. It lies within the scope of the invention to apply the skin-care agent to an absorbent article at the site which is regarded as being suitable for achieving satisfactory transfer of the agent to desired regions on the user.

In order to achieve satisfactory transfer, there are a number of conceivable mechanisms for regulating the release of the skin-care agent from the absorbent product. If the skin-care agent lies on the surface of the absorbent article, it is conceivable to cover the surface with a release paper for the purpose of avoiding undesirable migration of the skin-care agent during storage. It is also conceivable that external factors, for example heat, pressure, movement, shearing forces or moisture, affect the properties of the skin-care agent and can be used for effecting satisfactory transfer to the user during use.

In addition to applying a skin-care agent to the surface of the absorbent article in the form of a cream, as in the normal manner, the pectin can be present as film pieces, as described in the above experiment, which are placed on the surface layer, which faces the user, of the absorbent article. It is also conceivable to squirt or spray an aqueous solution of the pectin onto the surface layer in order to form discontinuous film drops or a thin continuous film when the solution dries.

A particularly interesting use of pectin is to employ it together with lactobacilli for the purpose of vitalizing the vulvovaginal environment in women. The ability of the pectin to moisten skin and mucous membranes makes it easier for the lactobacilli to adhere to the surfaces of the body tissues. The ability of the pectin to affect the pH and to function as an acidifier in the vulvovaginal environment in women having an elevated pH makes it easier for the lactobacilli to establish themselves and colonize the regions in question. It is conceivable to transfer the lactobacilli and the pectin simultaneously or for the pectin to be transferred first of all and for the lactobacilli to be transferred subsequently. One possibility is to use pectin as a carrier which is able to encapsulate the lactobacilli.

There are a number of advantages of using pectin as a skin-care agent for the mucous membranes. Apart from the fact that it has a moisture-preserving effect and can lower the pH, pectin is a well known substance which has been used previously on skin and mucous membranes and is preferred because of its good skin compatibility. By contrast, oestrogen, which is a hormone preparation which can be used for vitalizing and restoring dehydrated mucous membranes, suffers from certain known medical side-effects. Furthermore, pectin is a relatively cheap substance which can be produced from several different plant preparations. Because of its inherent gel-forming ability, the pectin can also readily interact with mucous membranes and skin and adheres well to these tissues when it comes into contact with them.

Even if, in this present description, the invention is primarily exemplified for absorbent articles for women, it is evident to the skilled person that pectin can nevertheless be used for other products such as babies' diapers, incontinence protections and sanitary towels where it is desirable to counteract dehydration and skin irritations or affect the pH and bring about an acidifying effect on the skin tissues or mucous membranes which are in contact with the product. These good skin-care properties result in pectin also being suitable for being used on incontinence products for elderly individuals, who frequently have a dry and fragile skin, which can become ulcerous and infected, or in diapers for infants, who run the risk of an irritated and dehydrated skin.

## Claims

1. An absorbent article, such as a sanitary towel, panty liner, tampon, diaper, interlabial product or incontinence protection, which is provided with a skin-care agent containing pectin which is intended to be transferred, during use, to the skin tissues or mucous membranes of the user, **characterized in that** the pectin is water-soluble and displays no amidation and **in that** a 1% (per cent by weight) aqueous solution of the pectin exhibits a pH of 34.

2. An absorbent article according to any one of the preceding claims, **characterized in that** the skin-care agent also contains lactobacilli.

3. An absorbent article according to any one of the preceding claims, **characterized in that** the skin-care agent is present in the form of a gel, film, liquid, cream, powder, emulsion, paste or fibre.

4. An absorbent article according to any one of the preceding claims, **characterized in that** the mean molecular weight of the pectin Is 20 000-400 000 u, preferably 50 000-150 000 u.

5. An absorbent article according to any one of the preceding claims, **characterized in that** the skin-care agent is intended to be transferred to skin, tissues and mucous membranes in the lower abdomen of a woman.

6. An absorbent article according to any one of the preceding claims, **characterized in that** the skin-care agent is located on or in a surface layer of the absorbent article which is facing the user during use.

7. An absorbent article according to claim 6, **characterized in that** the skin-care agent is located on the surface layer of the absorption body which is facing the user during use.

## Patentansprüche

1. Absorbierender Artikel, wie beispielsweise eine Binde, eine Slipeinlage, ein Tampon, eine Windel, ein interlabiales Produkt oder ein Inkontinenzschutz, welcher mit einem Pektin enthaltenden Hautpflegemittel versehen ist, welches während der Verwendung zur Übertragung auf das Hautgewebe oder die Schleimhäute des Benutzers vorgesehen ist, **dadurch gekennzeichnet, dass** das Pektin wasserlöslich ist und keine Amidierung zeigt, und dass eine 1 % (Gew.-%) wässrige Lösung des Pektins einen pH-Wert von 3-4 aufweist.

2. Absorbierender Artikel nach einem der vorgehenden Ansprüche, **dadurch gekennzeichnet, dass** das Hautpflegemittel auch Laktobazillen enthält.

3. Absorbierender Artikel nach einem der vorgehenden Ansprüche, **dadurch gekennzeichnet, dass** das Hautpflegemittel in Form eines Gels, einer Folie, einer Flüssigkeit, einer Creme, eines Pulvers, einer Emulsion, einer Paste oder einer Faser vorliegt.

4. Absorbierender Artikel nach einem der vorgehenden Ansprüche, **dadurch gekennzeichnet, dass** das mittlere Molekulargewicht des Pektins 20 000-400 000 u, vorzugsweise 50 000-150 000 u beträgt.

5. Absorbierender Artikel nach einem der vorgehenden Ansprüche, **dadurch gekennzeichnet, dass** das Hautpflegemittel zur Übertragung auf Hautgewebe und Schleimhauten im Unterbauch einer Frau vorgesehen ist.

6. Absorbierender Artikel nach einem der vorgehenden Ansprüche, **dadurch gekennzeichnet, dass** das Hautpflegemittel auf oder in einer während der Verwendung dem Benutzer zugewandten Oberflächenschicht des absorbierenden Artikels angeordnet ist.

7. Absorbierender Artikel nach Anspruch 6, **dadurch gekennzeichnet, dass** das Hautpflegemittel auf der während der Verwendung dem Benutzer zugewandten Oberflächenschicht des absorbierenden Artikels angeordnet ist.

## Revendications

1. Article absorbant, tel qu'une serviette hygiénique, un protège-slip, un tampon, une couche, un produit inter-labial ou une protection contre l'incontinence, qui est pourvu d'un agent de soin de la peau renfermant de la pectine et destiné à être transféré, lors de l'utilisation, aux tissus de la peau ou aux membranes muqueuses de l'utilisateur, **caractérisé en ce que** la pectine est soluble dans l'eau et ne présente aucune amidation, et **en ce qu'**une solution aqueuse de 1 % (pourcentage en poids) de la pectine présente une valeur pH de 3-4.

2. Article absorbant selon l'une quelconque des revendications précédentes, **caractérisé en ce que** l'agent de soin de la peau contient également des lactobacilles.

3. Article absorbant selon l'une quelconque des revendications précédentes, **caractérisé en ce que** l'agent de soin de la peau est présent dans la forme d'un gel, d'un film, d'un liquide, d'une crème, d'une poudre, d'une émulsion, d'une pâte ou d'une fibre.

4. Article absorbant selon l'une quelconque des revendications précédentes, **caractérisé en ce que** le poids moléculaire moyen de la pectine est 20 000-400 000 u, de préférence 50 000-150 000 u.

5. Article absorbant selon l'une quelconque des revendications précédentes, **caractérisé en ce que** l'agent de soin de la peau est destiné à être transféré aux tissus de la peau et aux membranes muqueuses dans la partie du bas-ventre chez la femme.

6. Article absorbant selon l'une quelconque des revendications précédentes, **caractérisé en ce que** l'agent de soin de la peau se trouve sur ou dans une couche de surface de l'article absorbant qui fait face à l'utilisateur pendant l'utilisation.

7. Article absorbant selon la revendication 6, **caractérisé en ce que** l'agent de soin de la peau se trouve sur la couche de surface du corps absorbant qui fait face à l'utilisateur pendant l'utilisation.
